# EUROPEAN PATENT SPECIFICATION

(11) **EP 3 365 001 B1**
(45) Date of publication and mention of the grant of the patent: **29.06.2022**
(21) Application number: 16787783.6
(22) Date of filing: 21.10.2016
(51) Int. Cl.: A61K 39/02

(54) **A VACCINE TO PROTECT AN MDA POSITIVE ANIMAL AGAINST A DISORDER ARISING FROM AN INFECTION WITH LAWSONIA INTRACELLULARIS**
IMPFSTOFF ZUM SCHUTZ EINES MDA-POSITIVEN TIERES GEGEN EINE ERKRANKUNG AUFGRUND EINER INFEKTION MIT LAWSONIA INTRACELLULARIS
VACCIN DESTINÉ À PROTÉGER UN ANIMAL POSITIF MDA CONTRE UN TROUBLE CAUSÉ PAR UNE INFECTION PAR LAWSONIA INTRACELLULARIS

(30) Priority: 22.10.2015 EP 15190969
(43) Date of publication of application: 29.08.2018
(73) Proprietor: Intervet International B.V., 5831 AN Boxmeer (NL)
(72) Inventor: JACOBS, Antonius, Arnoldus, Christiaan, 5831 AN Boxmeer (NL)
(74) Representative: Intervet International B.V.
(86) International application number: PCT/EP2016/075382
(87) International publication number: WO 2017/068126

(56) References cited:
- WO-A1-2015/082457
- WO-A1-2015/082458
- US-A1- 2007 014 815
- Niewiesk Stefan: "Maternal Antibodies: Clinical Significance, Mechanism of Interference with Immune Responses, and Possible Vaccination Strategies", Frontiers in Immunology, vol. 5, 15 September 2014 (2014-09-15), XP55896913, DOI: 10.3389/fimmu.2014.00446 Retrieved from the Internet: URL:https://www.ncbi.nlm.nih.gov/labs/pmc/ articles/PMC4165321/pdf/fimmu-05-00446.pdf >

## Description

### GENERAL FIELD OF THE INVENTION

The present invention pertains to a vaccine to protect an animal having maternally derived antibodies directed against *Lawsonia intracellularis,* against a disorder arising from an infection with *Lawsonia intracellularis.*

### BACKGROUND OF THE INVENTION

Proliferative enteropathy, also known as ileitis, is a common enteric disease of post-weaned pigs worldwide, caused by the obligate intracellular bacterium *Lawsonia intracellularis.* The characteristic lesion is a proliferation of immature enterocytes in the ileal intestinal crypts; these cells usually contain the causative bacteria within their apical cytoplasm. At autopsy, histologic lesions can be confirmed as Lawsonia-positive by visualization of 1.5 - 2.5 µm long, vibrioid shaped bacteria especially in enterocytes, but also often within macrophages located in the lamina propria between crypts, and in mesenteric lymph nodes. Clearance of the bacteria from the enterocytes leads to resolution of the associated proliferative lesions, indicating a direct local effect of the bacteria on the crypts (McOrist et al., Developed and resolving lesions in porcine proliferative enteropathy: possible pathogenetic mechanisms, Journal of Comparative Pathology, 115, 1996, pp 35-45). The presence of *Lawsonia intracellularis* in these lesions has been demonstrated using PCR, both in animals manifesting disease as in animals manifesting only subclinical infection. Clinical cases are usually present in the grower-finisher period; in some older finisher pigs an acute hemorrhagic form has been recorded.

Vaccines to combat *Lawsonia intracellularis* by inducing active protection are commercially available and described in the art,a dn for example available under the tradenames Enterisol^{®} Ileitis (Boehringer Ingelheim Vetmedica, USA) and Porcilis^{®} Ileitis (Merck Animal Health, USA). Maternally derived antibodies might interfere with these vaccines. WO 2015/082457 and WO2015/082458 describe the ID administration of inactivated Lawsonia antigens to protect against *Lawsonia intracellularis.* No protection through vaccination in the face of maternally derived antibodies is described. US20070014815 describes protection in the face of MDA's after intramuscular or oral administration.

### OBJECT OF THE INVENTION

It is an object of the invention to provide an alternative yet efficacious and safe vaccine for use in protecting an animal, in particular a pig, having maternally derived antibodies (MDA's) directed against *Lawsonia intracellularis,* against a disorder arising from an infection with *Lawsonia intracellularis.* It is a further object to provide a vaccine for use in such a method, which vaccine is able to better break through MDA's then a prior art vaccine.

### SUMMARY OF THE INVENTION

In order to meet the object of the invention, a vaccine as introduced in the "General field of the invention" section here above has been devised, the vaccine being for intradermal administration to the animal. In this respect it is noted that needle-less vaccination is not the same as intradermal vaccination. The World health Organization in its August 27, 2009 paper titled "Intradermal Delivery of Vaccines; A review of the literature and the potential for development for use in low- and middle-income countries" clearly indicates that "needle-less" vaccination does not necessarily mean "intradermal" vaccination (see Table 1, Page 3 of the review). Only when a needle-less device is "configured for intradermal vaccination", then a vaccine may indeed be delivered (at least partly) into the dermis. Otherwise the vaccine may be delivered subcutaneous or intramuscularly in its entirety.

The invention also enables the use of inactivated *Lawsonia intracellularis* antigen for the manufacture of a vaccine for intradermal administration to an animal that has maternally derived antibodies against *Lawsonia intracellularis,* to protect the animal against a disorder arising from an infection with *Lawsonia intracellularis.*

The same time, the invention enables a method for protecting an animal that has maternally derived antibodies against *Lawsonia intracellularis,* against a disorder arising from an infection with *Lawsonia intracellularis,* by intradermally administering a vaccine comprising inactivated *Lawsonia intracellularis* antigens.

It is noted that intramuscular vaccination with an inactivated *Lawsonia* vaccine has already been described in the art (see i.a. WO2009144088).This does not mean however that intradermal vaccination with such a vaccine obviously leads to an efficacious and safe vaccine, let alone to a vaccine that is able to break through maternally antibodies at least as good as when the vaccine is intramuscularly applied. The World Health Organization (WHO) for example has published an e-learning course called "Vaccine Safety Basics", in which course on page 53 it is reported that *"The route of administration is the path by which a vaccine (or drug) is brought into contact with the body. This is a critical factor for success of the immunization. A substance must be transported from the site of entry to the part of the body where its action is desired to take place. Using the body's transport mechanisms for this purpose, however, is not trivial."*

In this respect the California Department of Health Services' Immunization Branch has published guidelines for correct immunization (http://www.cdc.gov/vaccines/pubs/ pinkbook/ downloads/appendices/d/vacc_admin.pdf). With regard to the administration site it is stated on page 7, first full paragraph that *"The recommended route and site for each vaccine are based on clinical trials, practical experience and theoretical considerations. This information is included in the manufacturer's product information for each vaccine. There are five routes used in the administration of vaccines. Deviation from the recommended route may reduce vaccine efficacy or increase local adverse reactions."* On page 14 the only US-licensed intradermal vaccine is addressed: *"Fluzone Intradermal is the only U.S.-licensed vaccine that is administered by the intradermal route. It is approved only for use in persons 18 through 64 years of age. This Fluzone formulation is not the same* as *intramuscular formulations of inactivated influenza vaccine (TIV). Other TIV formulations should NOT be administered by the intradermal route."*

All in all, it is commonly known that vaccination at a particular site is not straightforward and requires experimentation to determine safety and efficacy.

It is noted that a vaccine in the sense of this invention is a constitution suitable for application to an animal, in general comprising one or more antigens such as attenuated or killed microorganisms and/or subunits thereof, or any other substance such as a metabolite of an organism, in an immunologically effective amount (i.e. capable of stimulating the immune system of the target animal sufficiently to at least reduce the negative effects of a challenge with wild-type micro-organisms), typically combined with a pharmaceutically acceptable carrier (*i.e.* a biocompatible medium, *viz.* a medium that after administration does not induce significant adverse reactions in the subject animal, capable of presenting the antigen to the immune system of the host animal after administration of the vaccine) such as a liquid containing water and/or any other biocompatible solvent or a solid carrier such as commonly used to obtain freeze-dried vaccines (based on sugars and/or proteins), optionally comprising immunostimulating agents (adjuvants), which upon administration to the animal induces an immune response for protecting an animal against a disorder that arises from an infection with a wild-type micro-organism, i.e. for aiding in preventing, ameliorating or curing such disorder.

In general, a vaccine can be manufactured by using art-known methods that basically comprise admixing the antigens (or a composition containing the antigens) with a pharmaceutically acceptable carrier, e.g. a liquid carrier such as (optionally buffered) water or a solid carrier such as commonly used to obtain freeze-dried vaccines. Optionally other substances such as adjuvants, stabilisers, viscosity modifiers or other components are added depending on the intended use or required properties of the vaccine.

Inactivated *Lawsonia intracellularis* antigen is any non-live *Lawsonia intracellularis* antigen derived from live *Lawsonia intracellularis* bacteria, whether or not recombinantly produced. In particular, inactivated *Lawsonia intracellularis* antigen, may comprise 1) killed bacteria, in particular killed whole bacteria, and/or (partly) lysed bacteria, together also called "bacterin", and/or 2) subunits such as proteins and other molecules typically produced by the corresponding live *Lawsonia intracellularis* bacteria.

In a first embodiment of the invention the vaccine induces protection after a single vaccination, i.e. no second (also called "booster") vaccination is required to arrive at an adequate level of protection. In a second embodiment of the invention the antigens comprise killed *Lawsonia intracellularis* bacteria (which may be in lysed or other non-whole cell form).

### EXAMPLES

### Introduction

The objective of this experiment was to test the efficacy of *Lawsonia* antigen, in particular freeze-dried *Lawsonia* bacterin, reconstituted in mineral oil adjuvant vaccine, and administered intradermally to pigs vaccinated and kept under field conditions, against *Lawsonia* challenge.

### Experimental design

At the age of 17weeks seventy five pigs (three groups of 25 pigs) were challenged orally with 20 ml homogenized *Lawsonia* infected intestinal mucosa. The pigs of group 1 had not been vaccinated (challenge controls). The pigs of group 2 had been vaccinated once intradermally in the neck (0.2 ml) with freeze-dried killed whole cell *Lawsonia* bacteria (such as known from WO20091444088 or the commercially available vaccine Porcilis^{®} Ileitis) reconstituted in mineral oil adjuvanted vaccine, at 3 weeks of age, using the commercially available intradermal vaccination device IDAL^{®} (available from MSD Animal Health, Boxmeer, The Netherlands). The pigs of group 3 had been vaccinated once intramuscularly in the neck (2 ml) with freeze-dried *Lawsonia* reconstituted in mineral oil adjuvanted vaccine, at 3 weeks of age, using a common syringe.

After challenge the pigs were daily observed for *Lawsonia* infection. At regular times before and after challenge the pigs were weighed and serum blood (serology) and faeces (Q-PCR) samples were collected. At 21 days after challenge the pigs were killed and post-mortem examined. The intestines were checked macroscopically for signs indicative for *Lawsonia intracellularis* infection. Ileum samples were collected for Q-PCR and (immuno)histological scoring (IHC).

### Material and methods

### Pre-mortem

One day before challenge, and on day 6, 13 and 20 after challenge, the pigs were weighed. Just before challenge, and on day 7 and 14 after challenge and during necropsy a faeces sample (gram quantities) of each pig was collected and cold stored until testing in a quantitative PCR (qPCR). Blood samples were collected just before challenge and 3w later (day of necropsy). The sera were stored frozen until the samples were tested in a *Lawsonia* antibody ELISA. Regarding the PCR, DNA was isolated from 0.2 g faeces and/or mucosa samples and a quantitative PCR on the AspA gene of *Lawsonia Intracellularis* was performed using the Kapa probe fast universal qPCR kit mastermix (Kapabiosystems).

### Post-mortem

Three weeks after challenge the pigs were euthanized and subjected to a post-mortem examination. The intestines, in particular the ileum, were examined for lesions indicative for *Lawsonia* infection. Ileum samples were collected for qPCR and immunehistological scoring (IHC). Gross scoring for the lesions was done by taking distal 50 cm of the small intestine (=ileum). The mucosa was scored as follows: 0=normal, 0.5=slight, 1=mild, 2=moderate, 3=severe thickening and/or reddening; 4= severe thickening and/or reddening with fibrin and/or necrosis. The percentage of the ileum affected by mucosal thickening and/or reddening was also scored (0-100%) and the total lesion score was ileum mucosa score x % ileum affected.

Regarding the histological scoring of ileum samples, a sample was collected from every animal (from affected area if present). Slides fixed and were stained with Hematoxylin-Eosin (HE stain) and with an immunohistochemical stain using *anti-Lawsonia intracellularis* Moab (IHC stain) and were examined microscopically. Scores were as follows. In the HE stain: 0=no abnormalities detected, 0.5=doubtful lesion, 1=mild lesions, 2=moderate lesions 3=severe lesions. IN the IHC stain: IHC stain: 0=no bacteria evident, 0.5=doubtful presence of bacteria, 1=presence of single/small numbers of bacteria within the slide, 2=presence of moderate numbers of bacteria within the slide, 3=presence of large numbers of bacteria within the slide. The total histological score = HE score x IHC score.

### Results

All control animals were infected as evidenced by qPCR, positive post-mortem scores and/or histological scores, evidencing a successful challenge. No adverse events that could be assigned to the vaccination were recorded. The vaccines can thus be regarded safe. Statistics were provided for the primary efficacy parameters, *viz.* qPCR on mucosa samples (infection), qPCR on faeces samples (shedding) and (immuno)histological ileum lesion scores. These parameters can be directly related to *Lawsonia* infection. For all tests the level of significance (α) was set at 0.05 and tests were two-sided.

On day of challenge (age 17w), 24 pigs of the control group were seronegative (i.e. <3.9 log2) and had an average titre of 3.0 log2. The pigs of groups 2 and 3 had an average titre of 5.7 and 5.5 log2, respectively. After the *Lawsonia* challenge all groups showed an increase in antibody titre. The pigs were obtained from a field study. From this study it became clear that many pigs at 3w of age, i.e. day of vaccination, had extremely high (maternally derived) antibody titres up to 11.7 log2 which is far above a titre which is typically obtained after vaccination (typically about 6-8 log2). To check whether the antibodies were indeed maternally derived, the respective sows were also blood sampled and tested for *Lawsonia* antibodies. The fact that there was a very good correlation between antibody titres of sows and piglets was a confirmation of the fact that the antibody titres of the piglets were of maternal origin. During the 14 weeks between vaccination and challenge the antibody titres in the control group decreased to seronegative. Despite the high maternally derived antibody titres both vaccinated groups showed clear antibody responses compared to the controls.

The data on average daily weight gain, PCR and post-mortem scores are indicated in table 1. The ADWG (in kg) was assessed in the third week post challenge since *Lawsonia* infection is at its peak around that time. PCR data are in log₁₀ pg DNA/µl.

**Table 1**

| **Group** | ADWG 3^{rd} week post ch. | PCR on faeces day 21 | PCR on mucosa day 21 | Mac. Ileum scores | Mic. Ileum scores |
|---|---|---|---|---|---|
| Control | 0.320 | 2.29 | 1.45 | 69.2 | 5.9 |
| ID | 0.640 | 0.88 | 0.39 | 20.4 | 2.2 |
| IM | 0.533 | 1.40 | 1.00 | 22.4 | 4.4 |

All primary parameters and the ADWG in the "ID" group were significantly distinct from challenge control. For the "IM" group this was the case for PCR on faeces (at day 21) and PCR on mucosa.

It appeared that despite the very high MDA level, vaccination worked well. ID vaccination seemed to be superior over IM vaccination given the better ADWG and overall lower scores.

## Claims

1. A vaccine comprising inactivated *Lawsonia intracellularis* antigen for use in a method to protect an animal that has maternally derived antibodies directed against *Lawsonia intracellularis,* against a disorder arising from an infection with *Lawsonia intracellularis,* by intradermal administration of the vaccine.

2. A vaccine for use according to claim 1, **characterised in that** the vaccine induces protection after a single vaccination.

3. A vaccine for use according to any of the preceding claims, **characterised in that** the antigens comprise killed *Lawsonia intracellularis* bacteria.

## Patentansprüche

1. Impfstoff, umfassend inaktiviertes *Lawsonia intracellularis-Antiqen*, zur Verwendung bei einem Verfahren zum Schutz eines Tiers, das von der Mutter stammende gegen *Lawsonia intracellularis* gerichtete Antikörper aufweist, gegen eine sich aus einer Infektion mit *Lawsonia intracellularis* ergebende Erkrankung durch intradermale Verabreichung des Impfstoffs.

2. Impfstoff zur Verwendung nach Anspruch 1, **dadurch gekennzeichnet, dass** durch den Impfstoff ein Schutz nach einer einzigen Impfung induziert wird.

3. Impfstoff zur Verwendung nach einem der vorhergehenden Ansprüche, **dadurch gekennzeichnet, dass** die Antigene abgetötete *Lawsonia intracellularis-*Bakterien umfassen.

## Revendications

1. Vaccin comprenant un antigène de *Lawsonia intracellularis* inactivé pour une utilisation dans un procédé de protection d'un animal qui possède des anticorps d'origine maternelle dirigés contre *Lawsonia intracellularis,* contre un trouble provenant d'une infection avec *Lawsonia intracellularis*, par administration intradermique du vaccin.

2. Vaccin pour une utilisation selon la revendication 1, **caractérisé en ce que** le vaccin induit la protection après une unique vaccination.

3. Vaccin pour une utilisation selon l'une quelconque des revendications précédentes, **caractérisé en ce que** les antigènes comprennent des bactéries *Lawsonia intracellularis* tuées.
